# EUROPEAN PATENT APPLICATION

(11) **EP 0 941 722 A1**
(43) Date of publication of application: **15.09.1999**
(21) Application number: 99200752.6
(22) Date of filing: 12.03.1999
(51) Int. Cl.: A61F 5/01

(54) **Knee Brace**

(30) Priority: 12.03.1998 NL 1008576
(71) Applicant: Inno- Brace B.V., 5926 SL Venlo (NL)
(72) Inventor: Jaspers, Pierre Joseph Theodor Marie, 3945 BE Cothen (NL)
(74) Representative: Duxbury, Stephen

(57) **Abstract**

Device for supporting the knee in order to prevent a subluxation reposition, the so-called "pivot shift", wherein the device comprises:
- a first frame element with a shape such that it fits around the rear side of the upper leg,
- a second frame element with a shape such that it fits around the front side of the lower leg,
- limiting means which extend from the first to the second frame element, which limiting means limit the displacement of the second frame element relative to the first frame element when the device is in position around a knee when the knee is straightened, which limiting means imitate the function of the anterior cruciate ligament of the knee joint.

## Description

The present invention relates to a device for supporting the knee and the use of such a device for this purpose.

The problems of instability at the knee in the case of a non-functioning anterior cruciate ligament are caused by the occurrence of a subluxation reposition movement during a loaded flexion. This abnormal movement, known as "pivot shift", occurs when the knee is moved under load, assuming a practically full extension. The "pivot shift" phenomenon occurs particularly in the four conditions specified below, i.e.:
- When landing on a virtually straightened knee after a jump and hyperextension of the knee in flexion,
- During sharp deceleration when running, wherein "pivot shift" can occur when coming down on the heel with a virtually straight leg,
- When wanting while running to make a rapid turning movement in the direction of the leg being used.
- When walking rapidly down a flight of stairs and landing on the lower step with an only slightly bent knee joint.

The "pivot shift" phenomenon occurs as a result of a reflexive tensioning of the quadriceps in order to decelerate the flexion. The tibia is then pulled forward by the patellar tendon and quadriceps and subluxates relative to the femoral condyle. This subluxation occurs because the tibia is not held back by the anterior cruciate ligament in the forward movement relative to the femoral condyle. When the loaded flexion continues there results an angular position between the tibia plateau and the horizon to ventral along which the femoral condyle can slide forward and is then caught by the patella. When this second movement is completed the initial subluxation of femoral condyle and tibial plateau is restored. This pattern of movement, which proceeds rapidly with a snapping movement, can result in injury to the knee joint in loaded situations. The best known of these injuries is the meniscal tear.

An object of the present invention is to provide a device which aims to prevent this subluxation reposition, the so-called "pivot shift".

According to a first aspect of the present invention a device is provided for supporting the knee in order to prevent a subluxation reposition, the so-called "pivot shift", wherein the device comprises:
- a first frame element with a shape such that it fits around the rear side of the upper leg,
- a second frame element with a shape such that it fits around the front side of the lower leg,
- limiting means which extend from the first to the second frame element, which limiting means limit the displacement of the second frame element relative to the first frame element when the device is in position around a knee when the knee is straightened, which limiting means imitate the function of the anterior cruciate ligament of the knee joint.

The inventor has therefore developed a device which substantially takes over the function of the anterior cruciate ligament at the beginning of the flexion from full knee extension. If the subluxation cannot occur in this situation, reposition sprain will obviously not occur either. The knee therefore substantially retains its normal pattern of movement during this loaded flexion.

Further features and details of the present invention can be found in claims 2 to 10.

A second aspect of the present invention relates to the use of the above stated device for supporting a knee.

A third aspect of the present invention relates to a mechanism for mutual fixing and allowing mutual movement of a femoral frame element and a tibial frame element.

A fourth aspect of the present invention relates to a knee-brace frame element with a shape such that it fits around the rear side of the upper leg.

A fifth aspect of the present invention relates to a knee-brace frame element with a shape such that it fits around the front side of the lower leg.

The present invention will now be described in more detail with reference to the figures, in which:
figure 1 is a perspective view of the present invention positioned around a knee,
figure 2 shows a side view of a preferred embodiment of the mechanism according to the present invention for mutual fixing of and allowing mutual movement of a femoral frame element and a tibial frame element of the device according to the present invention,
figure 3 is a side view of the device of figure 1,
figure 4 is a side view of the device of figures 1 and 3 in a second position, and
figure 5 shows a side view of a second preferred embodiment of the mechanism according to the present invention for mutual fixing of and allowing mutual movement of a femoral frame element and a tibial frame element of the device according to the present invention.

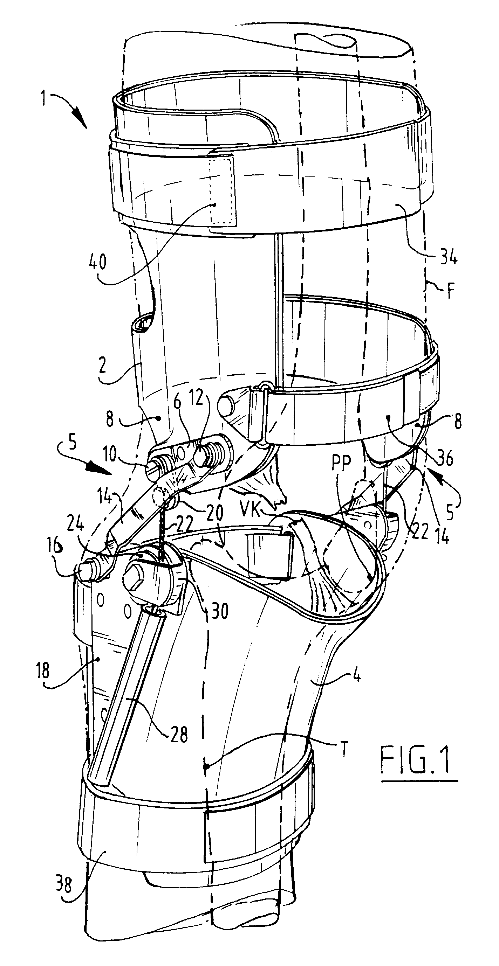

A device 1 according to the present invention (figure 1) comprises an upper shell 2, which is situated on the rear of the upper leg, and a lower shell 4 which is situated on the front side of the lower leg.

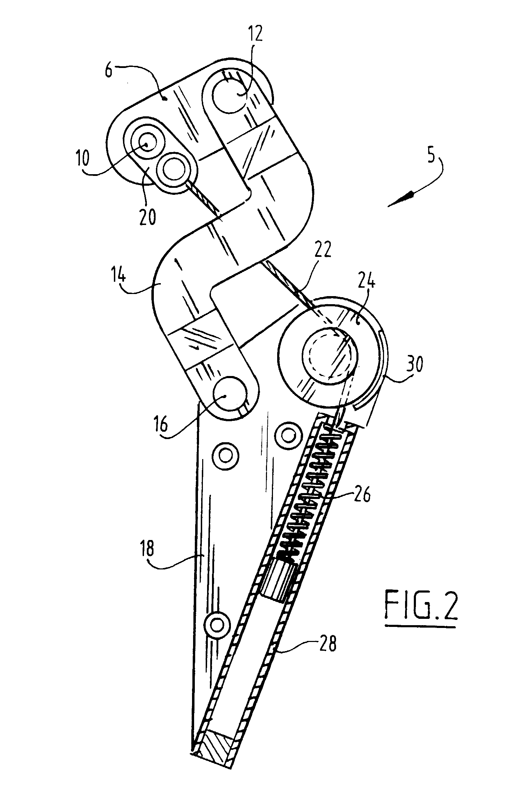

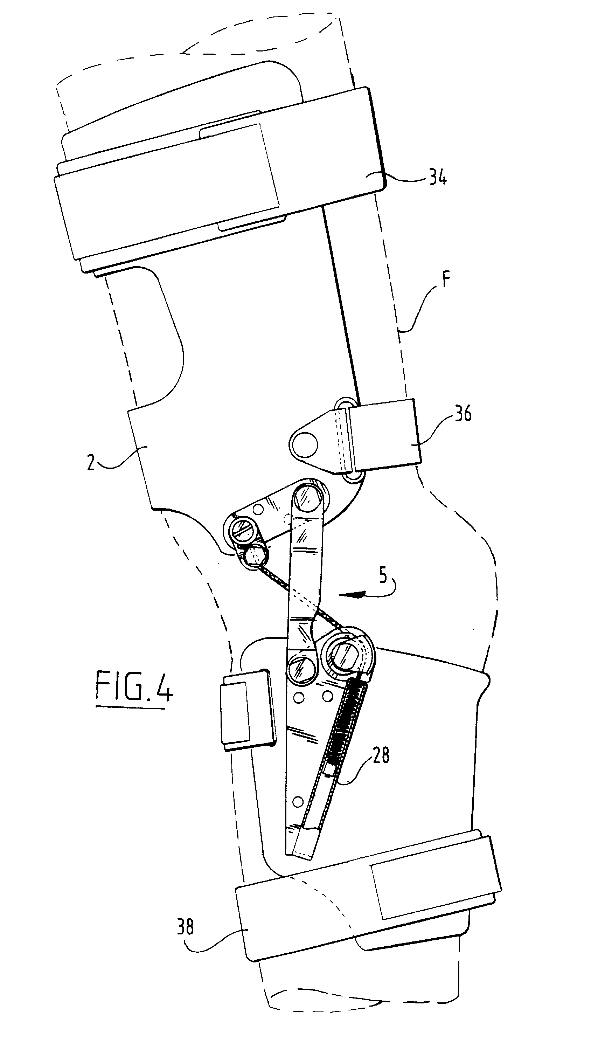

Device 1 has a mechanism 5 on each side (figure 4 and figure 1). Each mechanism 5 has a mounting plate 6 and a mounting frame 18. A metal connecting plate 14 is fixed to mounting plate 6 for rotation on an upper end thereof by means of a fastening pin 12. This connecting plate 14 is also fixed to mounting frame 18 for rotation on a lower end thereof. This connecting plate 14 can have a Z-shape in side view (figure 2) or a straight shape (figures 1, 3 and 4).

Mounting plates 6 run obliquely upward from back to front along two underside sections 8 of upper shell 2 and are fixed thereto by means of a fixing screw 10, which is arranged on the lower rear end of mounting plate 6, and fastening pin 12 which is arranged on the front upper end of mounting plate 6.

Fastening pin 12 extends between an opening (not shown) in the upper end of the metal connecting plate 14, whereby metal connecting plate 14 is rotatable around this fastening pin 12. Connecting plate 14 extends downward and rearward to a fastening pin 16 which extends through an opening (not shown) on a lower end of metal plate 14 and is secured to mounting frame 18 which is fixed to an upper side section of lower shell 4 (figure 1). Connecting plate 14 is thus also mounted rotatably on lower shell 4.

Fastening screw 10 runs through a fixing blade 20 to mounting plate 6.

Fastened to a front end of this fixing blade 20 is a cord 22, which is fastened at another end thereof around a running wheel 24 to a spring 26. This spring 26 (see figures 2-4) is arranged in a housing 28 which is also arranged on mounting frame 18. This housing 28 runs downward obliquely to the rear.

Running wheel 24 is provided with a protective guard 30.

During use the user pulls the device 1 over his leg until upper shell 2 is situated on the rear side of the upper leg and lower shell 4 is situated on the front side of the lower leg (figures 1, 3 and 4).

Lower shell 4 is provided with a hollow rear which proximally has a small transverse outward bulge 32 to dorsal which fits over the patellar tendon (PP). This outward bulge provides an exact location of lower shell 4 relative to the point of rotation of the knee.

Fastening straps 34, 36 of upper shell 2 and a fastening strap 38 of lower shell 4 can then be pulled tight by the user and secured round the upper leg respectively lower leg by means of velcro fastenings 40.

Since both upper shell 2 and lower shell 4 are mutually connected on the inside and outside of the knee by the two metal connecting plates 14 a rotation movement of the upper and lower shell 2 respectively 4 is possible relative to the two connecting plates 14 on an imaginary axis in a frontal plane.

Rotation of the upper and lower shells relative to each other is also possible via fastening pins 12 respectively 16.

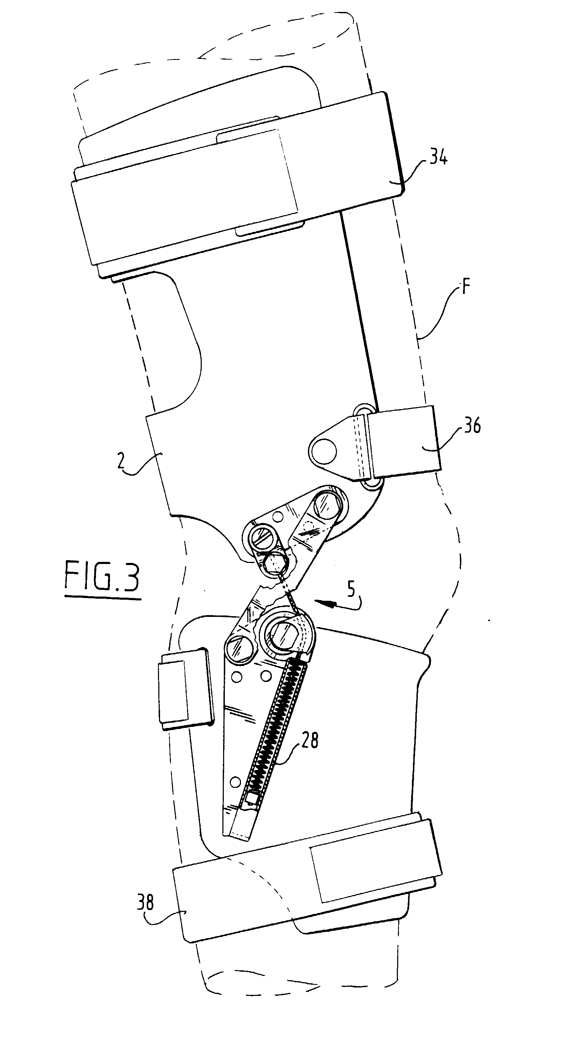

When a knee is bent the device 1 is positioned as shown in figure 3. In this situation the cord 22 is not tensioned and spring 26 is fully extended in housing 28.

In the case of reflexive tensioning of the quadriceps in order to decelerate flexion, the tibia T (figure 1) is pulled forward relative to the femur F (see figure 1) by the patellar tendon PP and quadriceps (not shown). When an anterior cruciate ligament VK is torn or sprained as shown in figure 1 with broken lines, the so-called "pivot shift" can occur.

This is prevented by the cords which are tensioned in the straightened position of the knee, whereby pulling forward of the tibia during deceleration does not occur, wherein lower shell 4 is not therefore moved forward. During this movement the cords 22 are tensioned around running wheels 24, as shown in figure 3, whereby springs 26 are compressed.

Metal connecting plate 14 ensures the mutual distance of the shells relative to each other in vertical direction. Cords 22 thus prevent subluxation of the tibia relative to the femur, since these cords limit the forward movement of the lower leg relative to the upper leg in the case of a straightened leg.

Cords 22 prevent shear in extension of lower shell 4 relative to upper shell 2 in horizontal direction in the case of a straightened knee.

When the knee is straightened, as is shown in figure 4, the cords run at an angle of about 45° relative to the joint gap of the knee. In this situation the cords run from the top at the rear to the bottom at front roughly parallel to an imaginary undamaged cruciate ligament (see figure 1).

On the rear top end the cords 22 are fastened to upper shell 2 by means of fixing blade 20 such that by placing this fixation point behind the point of rotation of the knee, the cords slacken when the knee is bent. During this slackening movement the lower shell 4 is moved rearward with the lower leg, whereby metal connecting plates 14 are rotated rearward around fastening pins 12, whereby mounting frame 18 is also moved rearward so that the cords 22 slacken.

Springs 26, which are preferably adjustable, enable this slackening of cords 22 to take place gradually.

The cord is fixed such that after arranging of the shell on a straightened knee it is tensioned maximally such that the spring reaches its extreme position. When the knee is bent and the shells thus rotate relative to each other, the tension in the cord is reduced as the tension on the spring decreases. Only when the spring is wholly slackened in a determined flexion position is the cord loose. In practice this latter will only occur with a substantial bending of the knee.

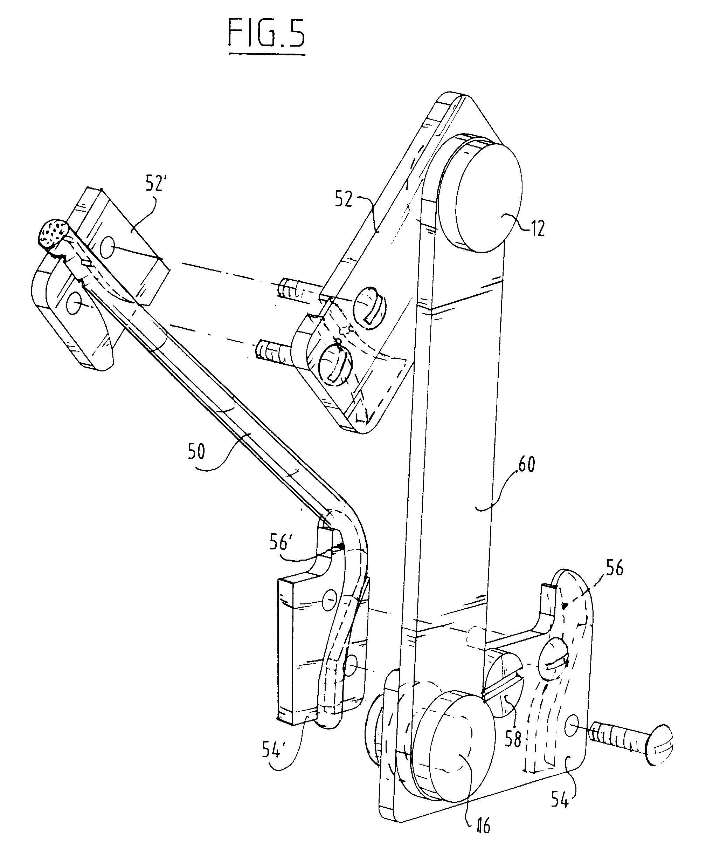

In a second embodiment of the present invention (fig. 5) cords 22 and springs 26 (see figures 1-4) are replaced by an elastic cord 50 with a finite elasticity which is comparable to the elasticity of spring 26 of figures 1-4. This elastic cord 50 is fixed to upper mounting plates 52, 52' and lower mounting plates 54, 54'.

Cord 50 no longer runs over a running wheel as in the embodiments shown in figures 1-4, but is guided along curves 56, 56' in the lower mounting plates 54, 54'. Similar curves are likewise arranged in upper plates 52, 52' so that cord 50 is not pulled round a sharp edge.

A stop pin 58 mounted on lower fixing plate 56 ensures that bracket 60 can never rotate further than vertical.

In contrast to the embodiment shown in figures 1-4, the bracket 60 in the second embodiment is a straight strip, since elastic cord 50 is now situated under mounting plates 52, 52', 54, 54'. This second embodiment provides a compact, efficient and elegant support of the knee with the purpose of preventing "pivot shift".

The invention is not limited to the above description; the rights sought are defined by the following claims.

## Claims

1. Device for supporting the knee in order to prevent a subluxation reposition, the so-called "pivot shift", wherein the device comprises:
- a first frame element with a shape such that it fits around the rear side of the upper leg,
- a second frame element with a shape such that it fits around the front side of the lower leg,
- limiting means which extend from the first to the second frame element, which limiting means limit the displacement of the second frame element relative to the first frame element when the device is in position around a knee when the knee is straightened, which limiting means imitate the function of the anterior cruciate ligament of the knee joint.

2. Device as claimed in claim 1, wherein the limiting means are alterable between a tensioned situation in which mutual repositioning of the two frame elements is substantially limited, and a non-tensioned situation.

3. Device as claimed in claim 1 or 2, wherein the limiting means are mounted in rotatable manner on the first frame element by means of rotatable mounting means.

4. Device as claimed in any of the foregoing claims, wherein the first and second frame elements are mutually connected by means of a connecting member, which member is arranged between the first and second frame elements.

5. Device as claimed in claim 4, wherein the connecting member is fixed at one end thereof to the first frame element by means of rotatable fastening means and is fixed at a second end thereof to the second frame element by means of rotatable fastening means.

6. Device as claimed in claim 5, wherein the rotatable fastening means of the limiting means are arranged on the first frame element with an interspacing relative to the connecting member.

7. Device as claimed in any of the foregoing claims, wherein the limiting means comprise a cord-like element.

8. Device as claimed in claim 7, wherein the connecting member comprises a metal plate.

9. Device as claimed in any of the foregoing claims, wherein the limiting means comprise a spring, which spring is fixed to the second frame element.

10. Device as claimed in claim 9, wherein the limiting means extend around a running wheel arranged on the second frame member.

11. Use of a device as claimed in any of the foregoing claims for supporting a knee.

12. Mechanism for mutual fixing of, allowing mutual movement of and mutual limiting of the movement of a first knee frame element with a shape such that it fits around the rear side of the upper leg and a second frame element with a shape such that it fits around the front side of the lower leg, which mechanism comprises:
- a connecting member which at one end thereof can be mounted rotatably on said first frame element and at a bottom side thereof can be mounted rotatably on said second frame element, and
- movement limiting means which at an upper end thereof can be mounted on the upper frame element and at a lower end thereof can be mounted on the lower frame element.

13. Knee-brace frame element with a shape such that it fits around the rear side of the upper leg.

14. Knee-brace frame element with a shape such that it fits around the front side of the lower leg.
